# EUROPÄISCHE PATENTSCHRIFT

(11) **EP 1 108 066 B1**
(45) Veröffentlichungstag und Bekanntmachung des Hinweises auf die Patenterteilung: **14.10.2009**
(21) Anmeldenummer: 99953632.9
(22) Anmeldetag: 27.08.1999
(51) Int. Cl.: C12Q 1/68

(54) **MIT NUKLEINSÄUREN BESCHICHTETE REAKTIONSRÄUME, VERFAHREN ZU IHRER HERSTELLUNG UND IHRE VERWENDUNG**
REACTION CHAMBERS COATED WITH DEFINED CONCENTRATIONS OF NUCLEIC ACIDS, METHOD FOR THE PRODUCTION AND USE THEREOF
RECIPIENTS DE REACTION TAPISSES D'ACIDES NUCLEIQUES, PROCEDE DE FABRICATION DESDITS ACIDES NUCLEIQUES ET LEUR UTILISATION

(30) Priorität: 28.08.1998 DE 19840531
(43) Veröffentlichungstag der Anmeldung: 20.06.2001
(73) Patentinhaber: AJ Roboscreen GmbH, 04129 Leipzig (DE)
(72) Erfinder: Koehler, Thomas, 04451 Borsdorf (DE)
(74) Vertreter: Wehlan, Helmut
(86) Internationale Anmeldenummer: PCT/DE1999/002715
(87) Internationale Veröffentlichungsnummer: WO 2000/012756

(56) Entgegenhaltungen:
- EP-A- 0 726 310
- DE-A- 19 716 154
- FR-A- 2 674 253
- STRATAGENE CATALOGUE, Seiten 274-277, XP002085450
- DAY I N M ET AL: "Dried template DNA, Dride PCR oligonucleotides and mailing in 96-well:LDL receptor gene mutation screening" BIOTECHNIQUES,US,EATON PUBLISHING, NATICK, Bd. 18, Nr. 6, 1995, Seiten 981-984-984, XP002085449 ISSN: 0736-6205
- SAMBROOK J. ET AL.,: "moleular cloning" 1987 , COLD SPRING HARBOUR PRESS, NEW YORK XP002132581 Seite 11.29 -Seite 11.30

## Beschreibung

Die Erfindung betrifft mit Nukleinsäuren beschichtete Reaktionsgefäße, Verfahren zu ihrer Herstellung durch die Beschichtung der Gefäße mit Standard-Nukleinsäuren, einen Testkit, enthaltend einen Standard-Strip, hergestellt unter Verwendung des erfindungsgemäßen Verfahrens, einen Satz von mindestens 2 Oligonukleotiden, die hierfür geeignet sind, einer Träger-Nukleinsäure sowie eine Vielzahl von Verwendungsmöglichkeiten des Verfahrens für den quantitativen Nachweis ausgewählter Nukleinsäuren in biologischen Substanzen.

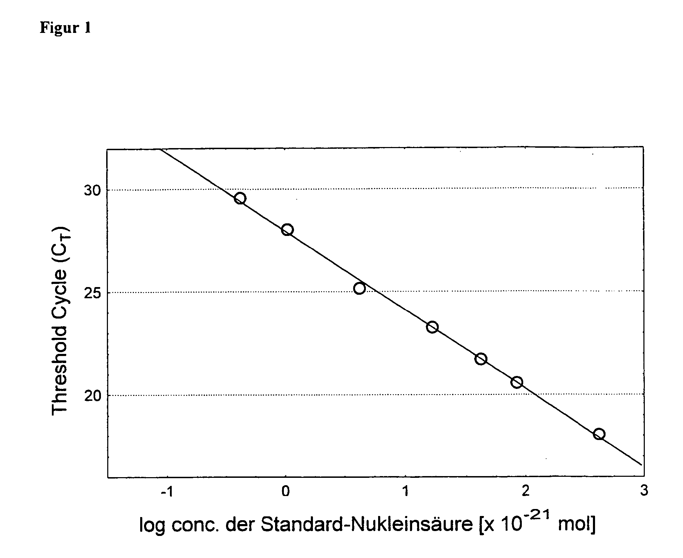

Viele Bereiche in der klinischen Forschung und Diagnostik, der pharmakologischen Wirkstoffprüfung sowie der Lebensmittelanalytik machen es erforderlich, Konzentrationen bestimmter Nukleinsäuren (Desoxyribonukleinsäure [DNA]- oder Ribonukleinsäure [RNA]) in einer zu analysierenden Probe genau zu kennen. Zur Messung insbesondere extrem geringer Analytkonzentrationen kommen häufig enzymatische Amplifizierungsverfahren zum Einsatz. Hierbei handelt es sich u.a. um die Verfahren Polymerasekettenreaktion (PCR, US Patente 4.683.195, 4.683.202, EP 0 200 362, EP 0 201 184; Hoffmann-La Roche), Ligasekettenreaktion (LCR, Abbott Diagnostics, North Chicago, IL, USA), Strand Displacement Amplification (SDA, Walker et. al. [1993], PCR Methods Appl. 3: 1-6, Becton-Dickinson Research Center) und Transcription-Mediated Amplification (TMA, Gen-Probe Inc., San Diego, CA), mit deren Hilfe bei extrem hoher Sensitivität Analytnukleinsäure-Konzentrationen gemessen werden können. Voraussetzung für den quantitativen Einsatz aller aufgeführten Technologien ist die Verfügbarkeit geeigneter synthetischer oder nativer Nukleinsäure-Standards exakt definierter Konzentration, die entweder als externe, d.h. in parallelen Ansätzen amplifizierte, oder interne Standards (d.h. simultan im selben Ansatz amplifizierte sog. Kompetitoren) verwendet werden. Während die Herstellung geeigneter Standards dem Fachmann bekannt ist (Zimmermann und Mannhalter 1996, Biotechniques 21: 268-279, Köhler et al. 1995, Quantitation of mRNA by polymerase chain reaction - Nonradioactive PCR methods. Heidelberg, Springer-Verlag), bestehen bislang ungelöste verfahrenstechnische Probleme bei der Überführung dieser Standards in eine anwendungsbereite und stabile Form, welches eine Grundvoraussetzung für die reproduzierbare Messung unbekannter Nukleinsäurekonzentrationen ist. Insbesondere bestehen Probleme bei der optimalen Lagerung hoch-verdünnter Nukleinsäuren. Diese beruhen im wesentlichen darauf, daß in der Praxis meist mit extrem niedrig konzentrierten Standard-Verdünnungsreihen gearbeitet wird (ca. 1 - 100000 Moleküle pro Reaktionsansatz), die trotz Lagerung bei Temperaturen zwischen -20°C und -80°C häufig instabil sind (Köhler et al. 1997, BioTechniques 23: 722-726). Diesem Problem wird u.a. so begegnet, daß niedrigkonzentrierte Nukleinsäure-Verdünnungen in der Form stabilisiert werden, daß der Verdünnung eine definierte Menge einer spezifischen Träger-Nukleinsäure zugesetzt wird, welche der zu detektierenden Nukleinsäuresequenz eine möglichst minimale Sequenzhomologie aufweist (De Kant et al. 1994, Biotechniques 17: 934-942, Köhler et al. 1997, BioTechniques 23: 722-726). Dies führt aber nicht immer zum Erfolg (siehe Figur 1), so daß allgemein empfohlen wird, alle erforderlichen Verdünnungsschritte ausgehend von einer Stammlösung definierter Konzentration tagtäglich neu durchzuführen. Dies wiederum ist aber mit dem Nachteil verbunden, daß die eingesetzten Standards arbeitsintensiv hergestellt werden müssen, variierender Pipettiergenauigkeit unterliegen und kostbare, verdünnte Chargen nur teilweise verbraucht werden können. Somit erhöhen sich zwangsläufig Kosten und Zeitaufwand bei gleichzeitig verringerter Reproduzierbarkeit und Zuverlässigkeit der Methodik. Anwendungstechnisch gesehen ist die geschilderte Verfahrensweise somit unökonomisch, mehreren Störgrößen unterworfen und demzufolge für diagnostische Routinelabors ungeeignet.

Day et al (Biotechniques, US, Eaton Publishing, Natick, Bd. 18, Nr. 6, 1995, Seiten 981-984) beschreiben die Anwendung von mit DNA beschichteten Gefäßen, wobei die Beschichtung mit zu untersuchenden DNA-Proben erfolgt. Nach Trocknung haften die adsorbierten DNA-Moleküle an der Innenseite des zur Beschichtung verwendeten Reaktionsraums. Die Zielstellung ist die qualitative Nukleinsäure-Diagnostik. Angaben zur Stabilität der DNA in den beschichteten Gefäßen werden nicht gemacht. Eine quantitative Nukleinsäurebestimmung ist mit dem Verfahren von Day et al nicht möglich.

Aufgabe der vorliegenden Erfindung ist es, Gefäße und Methoden zu entwickeln, mit denen die oben erwähnten Nachteile der Verfahren gemäß dem Stand der Technik vermieden werden können und die einfach in der Anwendung, über einen längeren Zeitraum bei gleichbleibender Qualität lagerbar und Bestandteile von Testkits sein können.

Die Aufgabe wurde durch die Beschichtung von Reaktionsräumen mit definierten Standard-Nukleinsäurekonzentrationen gelöst, welches folgende Teilschritte enthält:
- Herstellung und Reinigung geeigneter adsorbierbarer Nukleinsäure-Standards
- Bestimmung der exakten Konzentration des Produktes mittels High Performance Liquid Chromatography (HPLC), nachfolgend Kalibrierung genannt
- Herstellung einer Verdünnungsreihe aus dem kalibrierten Standard unter Zusatz definierter Mengen einer Träger-Nukleinsäure
- erfindungsgemäße Adsorption der Standard/Trägernukleinsäuregemische an für enzymatische Amplifizierungsreaktionen geeignete Reaktionsräume, so daß diese als Standards einsetzbaren, definiert beschichteten Gefäße in eine über lange Zeiträume haltbar Form überführt werden, ohne daß es zu Beeinträchtigungen der Qualität kommt.

Ebenfalls Gegenstand der Erfindung sind ein Satz von mindestens 2 Oligonukleotiden, ein Testkit entsprechend Erfordernissen eines Routinelabors und mehrere Verwendungsmöglichkeiten des Verfahrens.

Die nachfolgend beschriebene Erfindung stellt ein molekularbiologisches Verfahren dar, das eine Grundlage zur vorzugsweise automatisierten quantitativen Messung kleinster Mengen von Analytnukleinsäuren in diversen biologischen Materialien in Verbindung mit vorhergehender enzymatischer Amplifikation darstellt. Das erfindungsgemäße Verfahren besteht darin, Nukleinsäuren in eine zur Standardisierung quantitativer enzymatischer Amplifizierungsreaktionen geeignete Form zu überführen. Überraschenderweise hat sich herausgestellt, daß die erfindungsgemäße Methode die Herstellung von Nukleinsäurebeschichteten, sog. "ready-to-use" Standard-Reaktionsräumen erlaubt, die sich als einfach in der Anwendung erwiesen haben, die problemlos über einen langen Zeitraum bei gleichbleibender Qualität lagerbar und als Bestandteile von Testkits zu verwenden sind, somit den Bedürfnissen von Routinediagnostiklabors insbesondere in Hinblick automatisierter Analysen besser gerecht werden.

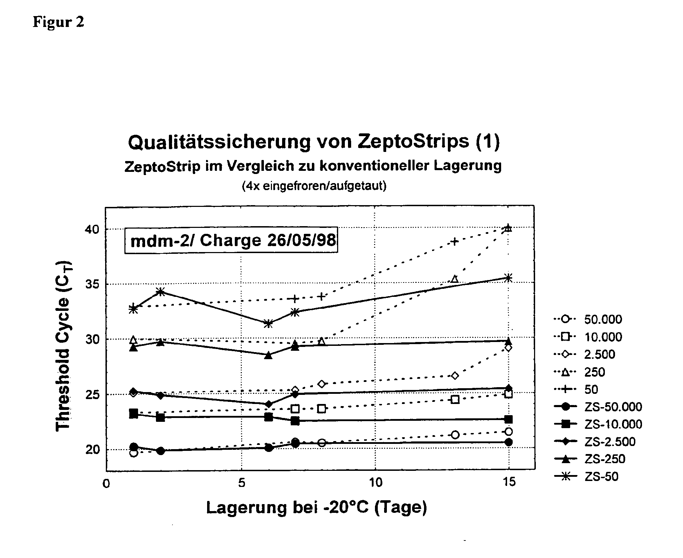

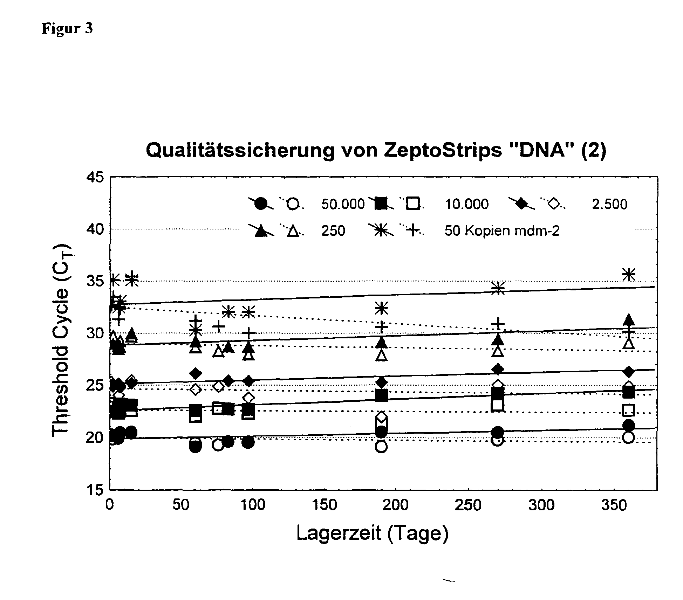

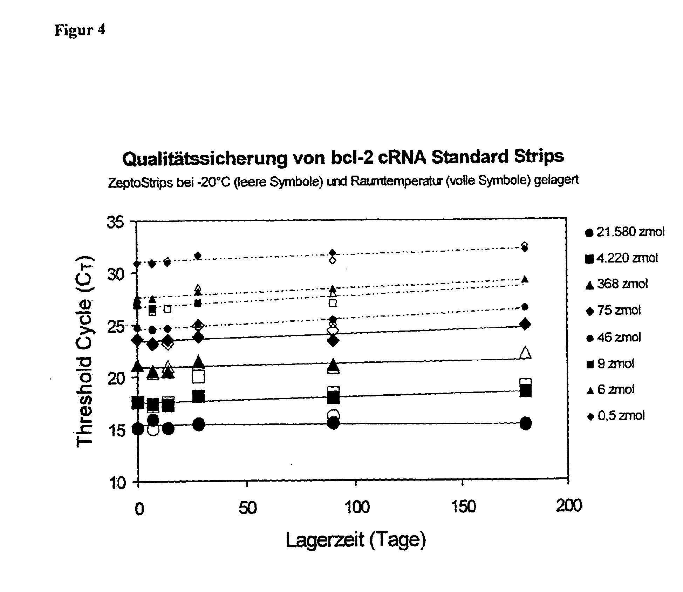

Nukleinsäuren im Sinne der Erfindung sind einzelsträngige oder doppelsträngige DNA, RNA, synthetische Analoga von DNA und RNA, sowie DNA, deren native Desoxy-Thymidin (dT)-Basen durch Desoxy-Uracil (dU) vollständig oder partiell ersetzt sind. Die Herstellung geeigneter Nukleinsäure-Standards erfolgt in einer dem Fachmann bekannten Form, vorzugsweise mit Hilfe der PCR, unter Nutzung spezifischer Primer-Oligonukleotide (Beispiel 1, Punkt 1A-B). Als Nukleinsäure-Standards werden native, auf enzymatischem Wege hergestellte Amplifizierungsprodukte oder synthetische Nukleinsäuren verwendet, deren Nukleotidsequenz einer zu bestimmenden Sequenz homolog ist, vorzugsweise identisch oder gekennzeichnet durch eine oder mehrere Punktmutationen, Deletionen oder Insertionen ist, die vorzugsweise außerhalb der Primer- und Sondenbindungsstellen liegen. DNA-Standards werden mittels enzymatischer Amplifikation von Target-Sequenzen, vorzugsweise mittels PCR, hergestellt, während RNA-Fragmente in bekannter Weise mittels *in vitro* RNA-Synthese unter Nutzung von RNA-Polymerasen (siehe Beispiel 2, Punkt 2.1., C) gewonnen werden können. Die hergestellten Nukleinsäurefragmente werden anschließend einer Reinigungsprozedur unterzogen, wobei DNA vorzugsweise mittels Agarosegelelektrophorese und anschließender Extraktion der Standard-Nukleinsäure aus dem Trenngel gereinigt wird (Beispiel 1, Punkt 1B), während *in vitro* hergestellte RNA in einer dem Fachmann bekannten Weise aus dem *in vitro* Sythese-Ansatz extrahiert wird (siehe Beispiel 2, Punkt 2.1., C). Die exakte Messung der Konzentration des gereinigten Nukleinsäure-Produktes erfolgt vorzugsweise mittels HPLC (Köhler et al. 1997, BioTechniques 23:722-726, Beispiele 1. Punkt 2). Anschließend wird von der kalibrierten Standard-Nukleinsäure eine Verdünnungsreihe hergestellt. Zur Verdünnung von DNA-Standards wird eine DNA-Lösung verwendet, die hergestellt wird, indem vorzugsweise DNA des Lambda-Phagen (z.B. Stamm: lambda cl 857 Sam 7, 48502 bp, Lambda-DNA) mittels 5 x 1-minütiger Ultraschall-Behandlung in Fragmente von ca. 1-2 Kilobasen (kb) überführt wird (Beispiel 1, Punkt 3; die durchschnittliche Fragmentlänge wurde mittels Agarosegelelektrophorese ermittelt). Dieser Schritt soll die verbesserte Adsorption während des Lyophilisationsprozesses bewirken und zu erhöhter Haltbarkeit der Standard-Nukleinsäure im Reaktionsraum beitragen. Es ist auch möglich, die Lambda-DNA unmodifiziert einzusetzen oder statt Lambda-DNA E. coli tRNA zu verwenden. Zur Verdünnung von RNA-Standards wird vorzugsweise eine Transport-RNA (tRNA)-Lösung verwendet (Köhler et al. 1995, Quantitation of mRNA by polymerase chain reaction - Nonradioactive PCR methods. Heidelberg, Springer-Verlag, Beispiel 2, Punkt 2.2.). Zur Quantifizierung eines Meßparameters werden verschiedene Standardverdünnungen hergestellt (Beispiel 1, Punkt 3, Beispiel 2, Punkt 2.2.), die vorzugsweise die Messung des gesamten physiologischen oder technologischen Konzentrationsbereiches des zu messenden Analyten erlauben. Hiervon werden Aliquote zur Beschichtung derjenigen Reaktionsräume verwendet, in welchen die zur Erstellung beispielsweise einer Eichkurve erforderlichen enzymatischen Nukleinsäureamplifikationen ablaufen sollen. Vorzugsweise werden 8 separate Reaktionsräume mit 8 verschiedenen Standardverdünnungen (sog. 8-er Strip) beschichtet. Erfindungsgemäß erfolgt die Beschichtung so, daß Aliquote der jeweiligen Standard-Nukleinsäure-Verdünnung supplementiert mit der Träger-Nukleinsäure direkt in den verwendeten Reaktionsräumen schonend getrocknet werden (Beispiel 1, Punkt 4, Beispiel 2, Punkt 2.3.). In einer besonders bevorzugten Weise erfolgt diese Lyophilisation unter Nutzung einer Vakuum-Zentrifuge oder einer Gefriertrocknungsanlage. In einer weiteren Form der Ausgestaltung erfolgt die Trocknung mittels eines gleichwertigen Trocknungsverfahrens, beispielsweise einem Verfahren zur überhitzungsfreien Produkttrocknung unter Einsatz von Mikrowellen (z.B. vertrieben über GWE mbH, Leuna). Die -wie beschrieben- hergestellten, beschichteten Reaktionsräume sind dadurch gekennzeichnet, daß die adsorbierten Nukleinsäure-Standards so fest an der Innenseite des zur Beschichtung verwendeten Reaktionsraumes haften, daß beispielsweise ein problemloser Versand auf dem Postweg gewährleistet werden kann. In den Figuren 1-4 ist beispielhaft dargestellt, welche Qualitätsanforderungen die mit dem erfindungsgemäßen Verfahren hergestellten Nukleinsäure-Standards erfüllen. Zum praxisrelevanten Test der auf dem beschriebenen Verfahren basierenden Produkte ist das derzeit beste, äußerst reproduzierbare Ergebnisse liefernde ABI PRISM™ 7700 Sequence Detection System (PE Applied Biosystems) eingesetzt worden. Mit diesem Detektionsautomaten lassen sich unter Nutzung des erfindungsgemäßen Verfahrens prinzipiell hoch reproduzierbare Eichkurven mit Korrelationen >-0.99 erstellen (Figur 1). Die erfindungsgemäß mit DNA beschichteten Reaktionsräume (Beispiel 1), z.B. die sog. "Optical" PCR Tubes, sind dem derzeitigen Stand der Technik überlegen, da diese im Vergleich zu herkömmlich eingesetzten PCR-Standards deutlich stabiler sind (Figur 2) und selbst bei Raumtemperatur über mindestens 1 Jahr ohne Qualitätsverlust lagerbar sind (Figur 3). Erfindungsgemäß mit in-vitro synthetisierter RNA beschichtete Reaktionsräume sind für mindestens 6 Monate sowohl bei - 20°C als auch Raumtemperatur stabil (Figur 4, Beispiel 2). Die beschichteten Reaktionsgefäße werden aufrecht stehend in einer geeigneten, mindestens 96 Gefäße aufnehmenden Träger-Box erfindungsgemäß mit einer selbsthaftenden Folie (z.B. Plastik- oder Aluminium-Folie, Parafilm) verschlossen, um eine Kontamination mit Fremd-Nukleinsäuren während Lagerung und Transport zu vermeiden. Je ein mit Folie verschlossener 8-er Strip, somit 8 verschiedene Konzentrationen der zur Beschichtung eingesetzten Nukleinsäuren enthaltend, wird als "ZeptoStrip" bezeichnet.

In den Reaktionsräumen können neben den kalibrierten Nukleinsäuren mindestens 2 spezifische als Primer bzw. Sonden fungierende markierte oder unmarkierte Oligonukleotide sowie weitere für die enzymatische Amplifikation erforderliche Reaktionskomponenten in lyophilisierter Form enthalten sein. Alternativ können verwendete Reaktionsräume auch alleinig die als spezifische als Primer bzw. Sonden fungierenden Oligonukleotide, die Träger-Nukleinsäure sowie weitere für die enzymatische Amplifikation erforderliche Reaktionskomponenten in lyophilisierter Form enthalten. Die erfindungsgemäßen Testkits bestehen aus mindestens einem "ZeptoStrip", mindestens 2 Oligonukleotiden sowie einer Träger-Nukleinsäure.

Das Wesen der Erfindung liegt in einer Kombination bekannter Elemente und neuer Lösungswege, die sich gegenseitig beeinflussen und in ihrer neuen Gesamtwirkung einen Gebrauchsvorteil und den erstrebten Erfolg ergeben, der darin liegt, daß nunmehr ready-to-use Standard-Reaktionsräume für den quantitativen Nachweis ausgewählter Nukleinsäuren in biologischen Substanzen zur Verfügung stehen.

Neben dem Abstellen der oben genannten Nachteile des Standes der Technik weist das erfindungsgemäße Verfahren somit eine Reihe von Vorteilen auf:
1. Ein manueller oder automatisierter Transfer von verdünnten, zur Standardisierung eingesetzten Nukleinsäuren in die verwendeten Reaktionskompartimente entfällt, da diese bereits im Reaktionsgefäß in lyophilisierter Form vorliegen. Damit erhöht sich wesentlich die Nutzerfreundlichkeit, da die sofort einsetzbaren Standard-Strips nur entnommen und in eine 96-well Trägerplatte eingesetzt werden müssen. Nach Zugabe der vorzugsweise vorgemischten Reagenzien für die nachfolgende Amplifizierungsreaktion sind keine weiteren Manipulationen mehr notwendig. Diese Vereinfachung erlaubt somit eine konsequente Automatisierung quantitativer enzymatischer Reaktionen.
2. Da nunmehr keine Pipettierung konzentrierter Standards mehr erfolgt, ist eine potentielle Kontaminationsquelle ausgeschaltet und somit die Gefahr falsch-positiver Ergebnisse deutlich verringert.
3. Durch Überführen der Standard-Nukleinsäuren in ein nicht-wäßriges Milieu wird a) potentiell ein unerwünschter mikrobieller Abbau der als Standards verwendeten Nukleinsäuren eingeschränkt bzw. verhindert, und b) eine Lagerfähigkeit selbst extrem niedrig konzentrierter Nukleinsäuren über längere Zeiträume selbst bei Zimmertemperatur erreicht (Figur 2-4). Diese Vorzüge vereinfachen ganz wesentlich sowohl Versand als auch Anwendung der auf diesem Verfahren beruhender Testkits.
4. Die verwendeten Träger-Nukleinsäuren verhindern eine unspezifische Standard-Adsorption an die zur Herstellung der Verdünnungsreihen eingesetzten Einweg-Materialien und sind -nachweisbar bei Zusatz zu einem PCR-Ansatz- gleichzeitig ein potenter "Enhancer" der enzymatischen Amplifikation.

Figur 1 zeigt eine typische Eichkurve, die mittels "real-time" PCR-Produktmessung am ABI PRISM™ 7700 Sequence Detection System unter Nutzung mit dem erfindungsgemäßen Verfahren hergestellter mdm-2 (murine double minute-2) Nukleinsäurestandards (Verwendung eines Standard-Strips, d.h. 8 verschiedener, lyophilisierter mdm-2 Standard-Nukleinsäurekonzentrationen) erhalten wurde (Beispiel 1, Punkt 5). Die errechnete Korrelationskoeffizient beträgt in diesem Fall -0.996.
Figur 2 zeigt in graphischer Form den Vergleich des erfindungsgemäßen Beschichtungsverfahrens und Nutzung der "real-time" Detektion wie in Figur 1 mit dem derzeitigen Stand der Technik. Herkömmliche, d.h. in wäßrigem Milieu gelagerte und am Versuchstag aliquotierte mdm-2 Standard-DNA Fragmente verschiedener Konzentration (50, 250, 2500, 10000 und 50000 Moleküle pro PCR-Ansatz), supplementiert mit Träger-DNA, wurden mit lyophilisierten Standards gleicher Konzentration verglichen. Während konventionell gelagerte Standards insbesondere sehr niedriger Konzentration (50 bzw. 250 Moleküle pro Ansatz) bereits nach 14 Tagen Lagerung und 4-maligen Einfrier-/Auftau-Zyklen vollständig abgebaut sind (äußert sich in der Abbildung durch Erreichen des Threshold Cycles 40, welcher der Amplifizierbarkeit 0 entspricht), ist bei Verwendung lyophilisierter Standards (ZeptoStrip) selbst bei niedrigsten verwendeten Nukleinsäurekonzentrationen kein Verlust feststellbar.
Figur 3 zeigt PCR-Resultate, die mit ZeptoStrips "mdm2-DNA" erhalten wurden, welche über einen Zeitraum von 1 Jahr alternativ entweder bei -20°C oder bei Zimmertemperatur gelagert wurden. Es ist zu erkennen, daß unabhängig von der Lagertemperatur identische PCR-Ergebnisse, d.h. weitgehend übereinstimmende C_{T}-Werte (d.h. parallele Kurven) erzielt wurden. Aus den Ergebnissen ist ersichtlich, daß die immobilisierte mdm2-DNA unabhängig von der Ansatzkonzentration und der Lagertemperatur über den gesamten untersuchten Zeitraum stabil bleibt.
Figur 4 zeigt TaqMan-PCR-Resultate, die mit ZeptoStrips "bcl2-cRNA" erhalten wurden, welche über einen Zeitraum von 6 Monaten alternativ entweder bei -20°C oder bei Zimmertemperatur gelagert wurden. Analog den Ergebnissen für mdm2-DNA war auch immobilisierte bcl2-cRNA unabhängig von der Ansatzkonzentration und der Lagertemperatur über den gesamten untersuchten Zeitraum stabil.

Die erfindungsgemäße Verwendung der mit Nukleinsäuren beschichteten Reaktionsgefäße liegt in Testkits zur Bestimmung ausgewählter Nukleinsäuren in biologischen Substanzen. Die Testkits bestehen aus mindestens einem mit Folie verschlossenen 8-er Strip, mindestens 2 Oligonukleotiden sowie einer Träger-Nukleinsäure.

Die folgenden Beispiele dienen der Verdeutlichung der Erfindung, ohne sie auf diese Beispiele zu beschränken.

### Ausführungsbeispiele

### Beispiel 1.

### Beschichtung von Polypropylen-Reaktionsräumen ("Optical Tubes") mit definierten Konzentrationen von doppelsträngiger mdm-2 Standard-DNA

### 1.1. Herstellung eines mdm-2 spezifischen Standard-DNA Fragmentes mittels PCR

### A. cDNA-Herstellung aus Gesamt-RNA, isoliert aus ADR5000 T-Lymphom-Zellinie (resistenzselektiert mit 5 µg Adriamycin pro ml Kulturmedium)

- 1 µg mittels RNAzol™ "B" (Tel-Test, Friendswood, TX, USA) gereinigte RNA in einem 20-µl Standard Reaktionsansatz, bestehend aus AMV Reverse Transcriptase Puffer (250 mmol/l Tris/HCl, pH 8.3; 250 mmol/l KCl, 50 mmol/l MgCl₂, 50 mmol/l Dithiothreitol, 2.5 mmol/l Spermidin), 5 U AMV Reverse Transcriptase, 0.5 mmol/l eines jeden dNTPs (Promega, Madison, WI, USA), 10 U rekombinanter RNase Inhibitor (AGS, Heidelberg, FRG), 200 ng Oligo (dT) (Amersham Pharmacia Biotech, Uppsala, Sweden), 1 Std. bei 42 °C in cDNA transkribieren

### B. PCR-Amplifizierung und Reinigung des Produktes

### ➢ PCR

- Je 2 µl-Aliquote der hergestellten cDNA werden in 6 identischen 50-µl Standard PCR Ansätzen bestehend aus 100 ng jedes 3' bzw. 5' Primers, 5 µl 10x Taq Polymerase Puffer (100 mmol/l Tris/HCl, pH 8.3; 500 mmol/l KCl, 15 mmol/l MgCl₂, 0.01% [wt/vol] Gelatine), 1.5 U AmpliTaq® Polymerase (Norwalk, CT, USA, Perkin-Elmer), und 8 µl dNTPs (0.2 mmol/l jedes Nukleotids unter Verwendung von dUTP anstatt dTTP) im GeneAmp®9600 Thermalcycler (Perkin-Elmer) amplifiziert.
- Programm: 94°C für 30 s, 55°C für 30 s, 72°C für 1 min 35 Zyklen
- Sequenzen der verwendete Amplifizierungsprimer (GenBank Accession Code für mdm-2: I25341)
   MDM2PR1 (1245-1264) 5'-GCC.AAG.AAG.ATG.TGA.AAG.AG-3'
   MDM2PR2 (1439-1455) 5'-ACT.GGG.CAG.GGC.TTA.TT-3'
- Länge des amplifizierten DNA-Fragmentes: 211 bp
   ➢

### ➢ Reinigung des 211 bp Fragmentes mittels Agarosegel-Elektrophorese

- 1.5 % Agarose-Gel (Easy-Cast™ Minigel, AGS, Heidelberg) herstellen, Gelslots mit 6-er Kamm, Kammer mit TAE-Puffer füllen (Submarine-Gel)
- die hergestellten PCR-Ansätze poolen und quantitativ auftragen
- Elektrophorese bei 100V, 45 min ausführen
- Ethidiumbromid-gefärbte Banden im UV-Licht sichtbar machen, möglichst genau und schnell mit Skalpell ausschneiden, in 1.5 ml-Eppendorfgefäße überführen
- DNA aus Gelblocks mittels QIAquick Gel Extraction Kit (Qiagen, Hilten) laut Vorschrift reinigen (Elution mit H₂O)

### 1.2. Kalibrierung der Standard-Stammlösung mittels HPLC

### ➢ HPLC-Equipment:

3-Line Degasser DG-980-50, PU-980 Intelligent HPLC Pump, Low Pressure Gradient Former, UV-975 UV/VIS Detector, AS-950 Intelligent Sampler, Column-Thermostat Jetstream 2 (Jasco Labor und Datentechnik GmbH, Gross-Umstadt, FRG).

### ➢ Stationäre Phase:

TSK DEAE-NPR Säule (4.6 mm ID, Länge: 35 mm) und DEAE-NPR Vorsäule (4.6 mm ID, Länge: 5 mm) (TosoHaas GmbH, Stuttgart, FRG)

### ➢ Mobile Phase:

Puffer A: 25 mmol/l Tris/HCl, 1 mol/l NaCl; pH 9.0
Puffer B: 25 mmol/l Tris/HCl; pH 9.0

### ➢ HPLC-Laufbedingungen:

- Druck: 80-120 bar (maximal 200 bar)
- Flußrate: 1 ml/min
- Temperatur: 20°C
- UV-Detektion bei 260 nm
- Analyt: ca. 10-200 ng gereinigtes PCR-Fragment, mit Puffer B auf 40 µl supplementieren, Injektion von 20 µl pro Lauf jeweils in Doppelbestimmung
- Standard (separater Lauf): 36 µl Puffer B plus 4 µl Low Mass DNA Ladder™ (Life Technologies, Gaithersburg, MD, USA), bestehend aus 6 glatt-endigen DNA-Fragmenten im Bereich 100 bis 2000 bp (Endkonzentration: 5 bis 100 ng DNA pro Bande), Injektion von 20 µl pro Lauf (Doppelbestimmung)
- Diskontinuierliches Gradientenprogramm über 25 min wie folgt durchführen:

| | |
|---|---|
| 1. Equilibrierung der Säule mit 25% Puffer A in Puffer B | |
| 2. 25% A in B: | Probenauftrag bis 0.5 min |
| 3. 25-43% A in B: | Linearer Gradient von 0.5-4.5 min |
| 4. 43-60% A in B: | Linearer Gradient von 4.5-20 min |
| 5. 60-100% A in B: | Linearer Gradient von 20-22 min |
| 6. 100-25% A in B: | Linearer Gradient von 22-24 min |
| 7.25% A in B: | Equilibrierung von 24-25 min |

Die Datengewinnung erfolgt mittels Integration der Peaks durch Borwin™ Chromatography Software, Version 1.20 (IMBS Developpements, Frankreich). Zur exakten Messung der Konzentration des gereinigten DNA-Standards wird die Fläche unter den individuellen Peaks ermittelt. Die unbekannte Konzentration der Standard-Nukleinsäure wird anhand der mittels Low Mass DNA Ladder™ erhaltenen Eichkurve berechnet.

### 1.3. Herstellung einer Standard-Verdünnungsreihe mit Träger-DNA

### Träger-Nukleinsäure:

10 OD Lambda (dam+) DNA (aus Bakteriophage lambda c1857 Sam7, AGS GmbH, Heidelberg), gelöst in 0.5 ml 10 mM Tris/HCl, pH 8.0; 1 mM EDTA, wird in 5x 1 min-Intervallen und zwischenzeitlicher Kühlung auf Eis mittels Ultraschall-Bad (Transsonic T570, Ultrasonics) bei maximaler Leistung in ca. 1-2 kb-Fragmente überführt, davon wird eine 10 ng/ml-Verdünnung hergestellt (1:100), im weiteren als Lambda-DNA bezeichnet.

### Herstellung der Standard-Verdünnungsreihe für mdm-2 (20x conc.)

| **Standard-No.** | **Verdünnung SL (Herstellung)** | **Endkonzentration [zmol/Ansatz]** | **Moleküle pro Ansatz** |
|---|---|---|---|
| **1** | 1: 10⁴ | 419.75 | 252.773 |
| **2** | 1: 10⁴ → 1:5 (20µl[No.1]+80µl λ) | 83.95 | 50.555 |
| **3** | 1: 10⁴ → 1:10 (10µl [No.1]+90µl λ) | 41.986 | 25.277 |
| **4** | 1: 10⁴ → 1:25 (10µl [No.3]+90µl λ) | 16.794 | 10.111 |
| **5** | 1: 10⁵ → (20µl [No.3]+180µl λ) | 4.199 | 2.528 |
| **6** | 1: 10⁶ → 1:4 (25µl [No.5]+75µl λ) | 1.050 | 632 |
| **7** | 1: 10⁶ → 1:10 (10µl [No.5]+90µl λ) | 0.420 | 253 |
| **8** | 1: 10⁶ → 1:50 (2µl[No.5]+98µl λ) | 0.084 | 51 |

| | | | |
|---|---|---|---|
| λ = Lambda-DNA, 10 ng/µl | | | |

Zur Herstellung der Standard-Gebrauchslösungen werden je 5 µl der Standard-Verdünnungsreihe in separate 1.7 ml Multi Twist Top Vials (Sorenson Bio Science, Salt Lake City, UT, USA; Vertrieb: Carl Roth GmbH.: Kat.-Nr.: 8184.1) pipettiert. Zur Herstellung multifunktioneller Strips (d.h. Strips, die zur sequentiellen oder simultanen quantitativen Messung mehrerer verschiedener Nukleinsäure-Sequenzen geeignet sind) werden weitere 19 Standards (jeweils 5 µl pro Standard-Nukleinsäure) zugesetzt und -wenn erforderliche- bis zu einem Endvolumen von 100 µl mit Lambda-DNA ergänzt.

### 1.4. Beschichtung der Reaktionsräume

- je 5 µl der unter Punkt 3 hergestellten Standard-Gebrauchslösungen in 8 verschiedene "optical tubes" (Fa. Perkin-Elmer, Kat.-Nr.: N8010935) pipettieren (von Position Al-A8 in abnehmender Konzentration), vorzugsweise diese Aliquotierung zur Verbesserung der Qualität mit einem Pipettier-Roboter (z.B. Biomek 2000, Fa. Beckman) ausführen
- Amplifikationsgefäße in schwarze Eppendorf Zentrifugen-Adapter (0.2 ml) einsetzen und Proben 30 min lang in Vakuumzentrifuge (z.B. Univapo 100 H mit Unijet Refrigerated Aspirator, Fa. UniEquip) bis zur völligen Trockene bei eingeschalteter Rotor-Gegenheizung lyophilisieren.

### 1.5. Test der hergestellten Strips mittels ABI PRISM™ 7700 Sequence Detection System

### optimierte mdm-2 TaqMan™-Methode:

| | | | |
|---|---|---|---|
| **Programm:** | 2-Step-PCR | 95°C | 10:00 min, anschließend |
| | 40 Zyklen | 95°C | 00:15 min |
| | | 60°C | 01:00 min |
| **Reaktionsbedingungen:** | 6 mM MgCl₂ | | |
| | 10 pM Primer mdm2Pr11 und mdm2Pr21 | | |
| | 2 pmol mdm2Probe | | |
| | 50 ng Lambda-DNA (5 µl) | | |
| | 2.5 U AmpliTaqGold™ | | |

dNTPs, Puffer aus TaqMan™ PCR Core Reagents Kit mit AmpliTaq™Gold

Ansatzvolumen: 50 µl

**verwendete Primer- und Sondensequenzen** (GenBank Accession Code für mm-2: I25341)
mdm2Pr11 (1295-1318) 5'-GAG.AGT.GTG.GAA.TCT.AGT.TTG.CCC-3'
mdm2Pr21 (1352-1373) 5'-TGC.AAC.CAT.TTT.TAG.GTC.GAC.C-3'

mdm2Probe (1320-1350)	5`-FAM-TTA.ATG.CCA.TTG.AAC.CTT.GTG.
TGA.TTT.GTC.A-XT-3`-TAMRA

### Beispiel 2.

### Beschichtung von Polypropylen-Reaktionsräumen ("Optical Tubes") mit definierten Konzentrationen von bcl2 Standard-copy RNA (cRNA)

### 2.1. Herstellung von bcl2 Standard cRNA, die sequenz-homolog zu nativer bcl2-mRNA ist

(nach einer modifizierten Methode von:: Grassi G, Zentilin L, Tafuro S, Diviacco S, Ventura A, Falaschi A, Giacca M. A rapid procedure for the quantitation of low abundance RNAs by competitive reverse transcription-polymerase chain reaction. Nucleic Acids Res 1994; 22: 4547-4549)

### A. Herstellung von bcl2 Template DNA mit inkorporiertem T7-Promoter

### ➢ Herstellung eines bcl2 Target-Fragmentes mit PCR1

- 100 ng CCRF ADR5000 cDNA (siehe Beispiel 1.1, A) wurden wie unter Beispiel 1.1, B in einem TRIO-Thermoblock™ 48 Thermal Cycler (Biometra, Göttingen) amplifiziert.
   Temperaturprofil PCR1: 94°C für 30 s, 55°C für 30 s, 72°C für 1 min 40 Zyklen
- Sequenzen der verwendeten Amplifizierungsprimer (GenBank Accession Code für bcl2: M14747)
   BCL2PR1 (3498-3516): 5'-CTT.TTG.CTG.TGG.GGT.TTT.G-3'
   BCL2PR2 (3896-3915): 5'-CTT.CTC.CTT.TTG.GGG.CTT.TT-3'
- Theoretische Länge des amplifizierten DNA-Fragmentes: 418 bp

### ➢ Inkorporation der T7-Promotersequenz in das synthetisierte bcl2-Target-Fragment mittels PCR2

**Tabelle 2/1: Pipettierschema für PCR2 (Herstellung von 6 identischen Ansätzen)**

| **Reaktionskomponenten** | **Ansatzvolumen µl** |
|---|---|
| H₂O (HPLC-rein) | 21,5 |
| 10x PCR-Puffer mit 1.5 mM MgCl₂ (PE Applied Biosystems) | 5 |
| dNTPs (je 1,25 mM, Promega/Boehringer Mannheim) | 8 |
| Primer bcl2-1T7 (50 ng/µl), verdünnt mit H₂O | 6 |
| Primer bcl2Pr2 (50 ng/µl), verdünnt mit H₂O | 2 |
| bcl2-Target Produkt (siehe 2.1. A, 1:1000 verdünnt mit H₂O) | 5 |
| AmpliTaq Gold (0,5 U/µl, PE Applied Biosystems) | 2,5 |

- Die Amplifizierung erfolgte nach folgendem Temperaturprofil: einmalig 95°C für 10 min, 40 Zyklen 95°C für 30 sec, 55°C für 30 sec, 72°C für 1 min, einmalig 72°C, 5 min, 4°C ∞
- Sequenzen der verwendeten Amplifizierungsprimer:
   BCL2PR2 (3896-3915): (siehe oben)
   bcl2-1T7: (unterstrichene Sequenz: T7-Promoter)
   5-cgg.gat.ccg.gat.cct.aat.acg.act.cac.tat.agg.gag.aCT.TTT.GCT.GTG.GGG.TTT.TG-3'
- Theoretische Länge des amplifizierten DNA-Fragmentes: 455 bp

### B. Reinigung und Kalibrierung des bclT7-DNA-Fragmentes

wie unter Beispiel 1.1. und 1.2. beschrieben

### C. In-vitro cRNA-Synthese (Herstellung von 3 identischen Ansätzen)

**Tabelle 2/2: Pipettierschema zur Herstellung der bcl2-cRNA-Syntheseansätze**

| **Reaktionskomponenten** | **Ansatzvolumen µl** |
|---|---|
| RNase-freies H₂O (mit DEPC behandelt) | 1 |
| 10x Transcription Buffer (Boehringer Mannheim) | 2 |
| ATP (20 mM, Amersham Pharmacia) | 1 |
| UTP (20 mM, Amersham Pharmacia) | 1 |
| GTP (20 mM, Amersham Pharmacia) | 1 |
| CTP (20 mM, Amersham Pharmacia) | 1 |
| gereinigtes bcl2-T7 dsDNA Fragment (6 ng/µl) | 10 |
| T7-RNA Polymerase (Boehringer Mannheim, 20U/µl) | 2 |
| RNase-Inhibitor (Amersham Pharmacia, 10 U/µl) | 1 |

- Die Inkubation der Ansätze erfolgte für 1 Std. bei 37°C im Thermoschüttler.
- Zur Erhöhung der cRNA-Ausbeute wurden nach Ablauf der Inkubation weitere 20 U T7 Polymerase pro Ansatz zugesetzt, danach wiederum Inkubation für 1 Std. bei 37°C (Thermoschüttier).
- Anschließend wurden alle drei Ansätze vereinigt und mit 60 U DNase I (RNase-frei, Boehringer Mannheim) für 50 min bei 37°C verdaut.
- Die Reinigung der cRNA erfolgte mittels konventioneller Phenol-Chloroform-Isoamylalkohol- (25:24:1) Extraktion wie in: Köhler T, Laßner D, Rost A-K, Thamm B, Pustowoit B, Remke H Eds.): Quantitation of mRNA by polymerase chain reaction - Nonradioactive PCR methods., Springer-Verlag Heidelberg, S. 36-37 beschrieben.
- Die Fällung der cRNA wurde für ca. 3 Std. bei -20°C durchgeführt, danach wurde 3x mit 300 µl 75% Ethanol gewaschen, zentrifugiert und das Pellett unter 100 µl 96%igem Ethanol über Nacht bei -20°C gelagert.
- Am nächsten Tag erfolgte die Trocknung des Pelletts in einer Vakuum-Zentrifuge (Univapo 100 H, UniEquip), das getrocknete Pellett wurde in 25 µl DEPC-H₂O gelöst, die Konzentrationsbestimmung erfolgte nach UV 260/280-Messung von 2x 2 µl-Aliquoten in 500 µl-Quartzküvetten.
- Die Gesamtausbeute aller 3 Ansätze betrug ca. 23 µg cRNA bei einem Ratio 260/280 >1,8.

### D. Charakterisierung der synthetisierten cRNA mittels nicht-denaturierender Agarosegel-Elektrophorese

(nach einer modifizierten Methode von: Collins ML, Zayati C, Detmer JJ, Daly B, Kolberg JA, Cha TA, Irvine BD, Tucker J, Urdea MS. Preparation and characterization of RNA standards for use in quantitative branched DNA hybridization assays. Anal Biochem 1995; 226:120-129)
- Eine Minigel-Kammer (Easy-Cast,™ AGS Heidelberg) wurde über Nacht mit 2%-iger Absolve NEF-971G Reinigungslösung (DuPont) RNase-frei gemacht, anschließend wurde 2x mit DEPC-H₂O gespült.
- Herstellung des Gels: 1%-iges Agarosegel (Qualex-Gold-Agarose, AGS) mit 1x TAE-Puffer (aus 50x Stammlösung mit DEPC-H₂O) herstellen (Ethidiumbromid [1,6 µl 10% EtBr] eingegossen), 10er Kamm; Laufpuffer: 1x TAE (4 µl EtBr/100 ml)
- Zur gereinigten und resuspendierten cRNA (ca. 1 µg pro 4 µl Volumen) sowie 4 µl einer 0,16-1,77 Kb RNA-Ladder (Gibco BRL) wurden 4 µl Formamid zugeben, und anschließend 5 min bei 65°C inkubiert.
- Danach erfolgte eine rasche Abkühlung auf Eis sowie anschließende Zugabe von 1 µl Gelladepuffer (RNase-frei) pro Tube, vom fertigen Ansatz werden 4 µl-Aliqote in die Testgel-Taschen pipettiert (Marker in die Außenslots, cRNA-Probe in die Innenslots).
- Die elektrophoretische Trennung wurde für ca. 2 Std. bei 80 V in der im Eisbad gekühlten, RNase-freien Minigel-Kammer durchgeführt. Eine Pufferzirkulation wurde durch intermittierende Bewegung des Laufpuffers in der Kammer erreicht.
- Die RNase-freie Dokumentation der Ergebnisse erfolgte mittels GelPrint Video Documentation Workstation (MWG-Biotech, Ebersberg) unter Nutzung von ONE-Dscan™ Software (Scanalytics, Billerica, MA, USA).
- Dem theoretischen Molekulargewicht der synthetisierten bcl2-cRNA von 418 b stand ein experimentell bestimmtes Molekulargewicht von ca. 400 b gegenüber.

### 2.2. Herstellung einer bcl2-cRNA-Verdünnungsreihe

Die Verdünnung der gereinigten bcl2-cRNA erfolgte mit E.coli tRNA-Lösung (100 ng/µl DEPC-H₂O, Boehringer Mannheim).

**Tabelle 2/3: Herstellung einer bcl2-cRNA-Verdünnungsreihe nach folgendem Schema:**

| **Standard Nr.** | **cRNA-Verdünnungsfaktor** | **RNA-Konzentration (Angabe in zmol pro 5 µl verdünnter Lösung)** |
|---|---|---|
| 1 | 1:10³ | 1610 |
| 2 | 1:10⁴ | 161,05 |
| 3 | 1:10⁵ | 16,105 |
| 4 | 1:10⁵→ 1:5 | 3,22 |
| 5 | 1:10⁶ | 1,6105 |
| 6 | 1:10⁶→ 1:5 | 0,322 |
| 7 | 1:10⁷ | 0,16105 |
| 8 | 1:10⁸ | 0,016105 |

### 2.3. Beschichtung von 96 "Optical Tubes" (1 Platte) mit bcl2 Standard-cRNA definierter Konzentration

- Von jeder bcl2-cRNA-Verdünnung (siehe Tabelle 2/3) wurden 150 µl hergestellt und in ein bereitgestelltes, kühlbares Rack einer Biomek®2000 Pipettierworkstation (Beckman Instruments Inc., Fullerton, CA, USA) positioniert.
- Eine 96-well Trägerplatte wurde mit 96 "Optical Tubes" beschickt und ebenfalls in die dafür vorgesehene Position der Workstation gesetzt.
- Mit Hilfe des Roboters und unter Nutzung geeigneter, gestopfter Einweg-Pipettenspitzen wurden je 5 µl von Standard "1" in die "Optical Tubes" der Position A1-A12, von Standard "2" in Tubes B1-B12, von Standard "3" in Tubes C1-C12 usw. pipettiert.
- Die Standard-cRNA enthaltenen Gefäße wurden anschließend komplett für 30 min bei - 80°C schockgefroren und unter Vermeidung eines zwischenzeitlichen Auftauens sofort in einer vorgekühlten Lyovac GT2 (AMSCO Finn-Aqua GmbH, Hürth) für 1 Std. lyophilisiert.
- Anschließend wurden die Tubes mit einer selbstklebenden Folie (z.B. Biomek™ Seal & Sample Aluminium-Folien, Beckman Instruments) manuell verschlossen. Die ZeptoStrips "RNA" wurden generiert, indem die an den Tubes anhaftende Folie vertikal so geschnitten wurde, daß zusammenhängende Strips der Positionen A1-H1, A2-H2, A3-H3 usw. entstanden, welche jeweils eine Konzentration einer jeden bcl2-cRNA Verdünnung enthielten.
- Von den nunmehr hergestellten 12 Strips wurden je 6 Strips alternativ entweder bei -20°C oder bei Zimmertemperatur über den untersuchten Zeitraum gelagert

### 2.4. Analyse der hergestellten bcl2 cRNA Strips mittels ABI PRISM^{TM} 7700 Sequence Detection System

### A. Materialien:

- 5x TaqMan EZ Puffer: 250 mM Bicine, 575 mM K-acetate, 0.05 mM EDTA, 300 nM ROX (6-carboxy-tetramethyl-rhodamin), 40% (w/v) Glycerol; pH 8.2
- 25 mM Mn(OAc)₂
- dNTPs: dATP (10 mM), dCTP (10 mM), dGTP (10 mM) und dUTP (20 mM) im Volumenverhältnis 1:1:1:1 gemischt

### B. Sequenzen der verwendeten Oligonukleotide

bcl2Pr1 (3498-3516) wie oben
bcl2Pr21 (3572-3591) 5'-GCA.AGT.GCA.GCC.ACA.ATA.CT-3' 
bcl2Sonde (3547-3568)    5`-FAM-CAG.TTC.TGG.GGC.CAA.GAG.GCT.GTXT-3`-
TAMRA
(FAM = 6-carboxyfluorescein, TAMRA = 6-carboxytetramethylrhodamine)

### C. Kombinierte Reverse Transcription und PCR (RT-PCR) unter Nutzung des Enzyms rTth Polymerase (PE Applied Biosystems)

- Am jeweiligen Versuchstag wurden in je einen der alternativ bei -20°C oder Raumtemperatur gelagerten bcl2-cRNA Strips folgende Reaktionskomponenten pipettiert (Tabelle 2/4, S = Standard-Nr., Ansatzvolumen: 50 µl)

**Tabelle 2/4: PCR3**

| **Reaktionskomponente** | **S1** | **S2** | **S3** | **S4** | **S5** | **S6** | **S7** | **S8** |
|---|---|---|---|---|---|---|---|---|
| DEPC-H₂O | 10.5 | 10.5 | 10.5 | 10.5 | 10.5 | 10.5 | 10.5 | 10.5 |
| 5x EZ-Puffer mit ROX | 10 | 10 | 10 | 10 | 10 | 10 | 10 | 10 |
| dNTPs für TaqMan | 6 | 6 | 6 | 6 | 6 | 6 | 6 | 6 |
| bcl2Pr1 (50 ng/µl) | 1 | 1 | 1 | 1 | 1 | 1 | 1 | 1 |
| bcl2Pr21 (50 ng/µl) | 1 | 1 | 1 | 1 | 1 | 1 | 1 | 1 |
| bcl2-Sonde (0,79 pmol/µl) | 2.5 | 2.5 | 2.5 | 2.5 | 2.5 | 2.5 | 2.5 | 2.5 |
| Mn (OAc)₂, 25 mM | 5 | 5 | 5 | 5 | 5 | 5 | 5 | 5 |
| rTth-Polymerase (PE Applied Biosystems, 2.5 U/µl) | 2 | 2 | 2 | 2 | 2 | 2 | 2 | 2 |

Temperaturprofil am ABI PRISM™ 7700 Sequence Detection System:
59°C, 30 min (Reverse Transcription)

| | |
|---|---|
| | 95°C, 5 min |
| 40 Zyklen | 95°C, 15 sec |
| | 59°C, 1 min |

- Mittels PCR3 (Tabelle 2/4) wurde die Stabilität von erfindungsgemäßen bcl2-cRNA Strips nach jeweils 7, 14, 28, 90 und 180 Tagen Lagerung (sowohl -20°C als auch Raumtemperatur) analysiert. Die Ergebnisse sind in Figur 4 zusammengefaßt.

### Legende zu den Figuren

Figur 1:
   Charakteristische Eichkurve, erstellt mittels PCR-Produkt Messung am ABI PRISM™ 7700 Sequence Detection System unter Verwendung eines "ZeptoStrips", welcher mit dem erfindungsgemäßen Verfahren mit mdm-2 DNA beschichtet wurde, sowie der zugehörigen Methode (siehe Ausführungsbeispiel 1, Punkt 5), r = -0.996.
Figur 2:
   Vorteile von ZeptoStrips (ZS) im Vergleich zu Standard-Nukleinsäuren, die nach derzeitigem Stand der Technik konventionell im wäßrigen Milieu gelagert wurden. Gestrichelte Linien: konventionelle Lagerung, durchgezogene Linien: ZeptoStrip. Legende: Initiale Zahl von Nukleinsäuremolekülen per PCR-Ansatz.
Figur 3:
   Lagerfähigkeit von ZeptoStrips mdm2-DNA über einen verfolgten Zeitraum von 1 Jahr ohne Qualitätsverluste. Legende: Initiale Zahl von Nukleinsäuremolekülen pro PCR-Ansatz. Durchgezogene Linien: Lagerung bei +25°C, gestrichelte Linien: Lagerung bei -20°C.
Figur 4:
   Stabilität von ZeptoStrips "bcl2-cRNA", die über einen Zeitraum von 6 Monaten alternativ entweder bei -20°C oder bei Zimmertemperatur gelagert wurden. Dargestellt sind die für jede Standard-Konzentration berechneten Regressionsgeraden unter Berücksichtigung aller jeweils bei -20°C und +25°C-Lagerung erhaltenen Meßwerte. Legende: Konzentration der im Reaktionsraum immobilisierten bcl2-cRNA in zeptomol pro Ansatz.

## Patentansprüche

1. Mit nativen, synthetisch oder enzymatisch hergestellten Nukleinsäuren beschichtete Reaktionsräume, die bei Raumtemperatur ohne Qualitätsverlust lagerbar sind, erhältlich **dadurch**, dass die Beschichtung mit kalibrierten Standard-Nukleinsäuren unter Zusatz von definierten Mengen einer Träger-Nukleinsäure auf nicht-kovalentem Wege an chemisch oder biochemisch nicht-modifizierte Oberflächen der Innenwandung von Reaktionsräumen durch schonende Trocknung erfolgt.

2. Reaktionsräume nach Anspruch 1, **dadurch gekennzeichnet, dass** diese aus Glas- oder Plastik-Gefäßen oder aus Glaskapillaren bestehen.

3. Reaktionsräume nach den Ansprüchen 1 und 2, **dadurch gekennzeichnet, dass** als Standard-Nukleinsäuren DNA, RNA, synthetische Analoga von DNA und / oder RNA sowie dU-haltige DNA eingesetzt werden.

4. Reaktionsräume nach den Ansprüchen 1 bis 3, **dadurch gekennzeichnet, dass**
a) zur Verdünnung von DNA-Standards eine DNA-Lösung eingesetzt wird, welche eine minimale Sequenzhomologie zu dem zu analysierenden Nukleinsäuregemisch aufweist
b) zur Verdünnung von RNA-Standards eine tRNA-Lösung verwendet wird.

5. Reaktionsräume nach den Ansprüchen 1 bis 4, **dadurch gekennzeichnet, dass** als Träger-Nukleinsäure DNA des Lambda-Phagen verwendet wird, welche zuvor mittels Ultraschall-Behandlung in leicht desorbierbare Fragmente einer mittleren Länge von ca. 1-2 kb überführt wird.

6. Verfahren zur Herstellung der Reaktionsräume nach den Ansprüchen 1 bis 5, **dadurch gekennzeichnet, dass** kalibrierte Standard-Nukleinsäuren und definierte Mengen zugesetzter Träger-Nukleinsäure direkt in zur enzymatischen Amplifikation geeignete Reaktionsräume aliquotiert werden und anschließend nicht-kovalent direkt an die Innenwandung des Reaktionsraumes durch Lyophilisierung mittels Gefriertrocknung oder Vakuum-Zentrifugation adsorbiert werden.

7. Verfahren nach Anspruch 6, **dadurch gekennzeichnet, dass** Plastik-Gefäße bzw. Glaskapillaren beschichtet werden.

8. Verfahren nach den Ansprüchen 6 und 7, **dadurch gekennzeichnet, dass** als Nukleinsäuren DNA, RNA, synthetische Analoga von DNA und / oder RNA sowie dU-haltige DNA eingesetzt werden.

9. Verfahren nach den Ansprüchen 6 bis 8, **dadurch gekennzeichnet, dass**
a) zur Verdünnung von DNA-Standards eine DNA eingesetzt wird, welche eine minimale Sequenzhomologie zu dem zu analysierenden Nukleinsäuregemisch aufweist und
b) zur Verdünnung von RNA-Standards eine tRNA-Lösung verwendet wird.

10. Verfahren nach den Ansprüchen 6 bis 9, **dadurch gekennzeichnet, dass** als Träger-Nukleinsäure DNA des Lambda-Phagen verwendet wird, welche zuvor mittels Ultraschall-Behandlung in leicht desorbierbare Fragmente einer mittleren Länge von ca. 1-2 kb überführt wird.

11. Verfahren nach den Ansprüchen 6 bis 10, **dadurch gekennzeichnet, dass** entsprechende Reaktionsräume simultan mit einer Vielzahl (mindestens 2) diverser, Analytsequenzspezifischer, kalibrierter Nukleinsäuren, ggf. unterschiedlichem zellulären oder organischen Ursprungs bzw. aus verschiedenen Spezies stammend, beschichtet werden.

12. Verfahren nach den Ansprüchen 6 bis 11, **dadurch gekennzeichnet, dass** die Beschichtung von mindestens 96, im Mikrotiterplatten-Format angeordneten Reaktionsräumen mit mindestens 12 x 8 sequenzspezifischen Standard-Nukleinsäuren abnehmenden, den gesamten erwarteten Konzentrationsbereich der zu messenden Analytnukleinsäure erfassenden Konzentrationen (höchste Konzentration: A1-12, niedrigste Konzentration: H1-12) erfolgt.

13. Verfahren nach den Ansprüchen 6 bis 12, **dadurch gekennzeichnet, dass** die beschichteten Reaktionsräume aufrecht stehend in einer geeigneten, mindestens 96 Gefäße aufnehmenden Träger-Box verschlossen werden.

14. Verfahren nach den Ansprüchen 6 bis 13, wobei in den Reaktionsräumen neben den kalibrierten Nukleinsäuren mindestens zwei spezifische als Primer bzw. Sonden fungierende markierte oder unmarkierte Oligonukleotide, die Träger-Nukleinsäure sowie weitere für die enzymatische Amplifikation erforderliche Reaktionskomponenten in lyophilisierter Form enthalten sind oder spezifische als Primer bzw. Sonden fungierende markierte oder unmarkierte Oligonukleotide, die Träger-Nukleinsäure sowie weitere für die enzymatische Amplifikation erforderliche Reaktionskomponenten in separaten Gefäßen ohne Nukleinsäure-Standard in lyophilisierter Form enthalten sind.

15. Verwendung von mit Nukleinsäuren beschichteten Reaktionsräumen nach den Ansprüchen 1 bis 5 in Testkits zur Bestimmung ausgewählter Nukleinsäuren in biologischen Substanzen.

16. Verwendung nach Anspruch 15, **dadurch gekennzeichnet, dass** die Testkits aus mindestens einem mit Folie verschlossenen ZeptoStrip (8-er Strip aus verschlossenen, mit 8 verschiedenen Nukleinsäure-Konzentrationen beschichteten Reaktionsräumen), mindestens zwei Oligonukleotiden sowie einer Träger-Nukleinsäure bestehen.

## Claims

1. Reaction chambers coated with native, synthetically or enzymatically prepared nucleic acids, which are storable without quality downslide at room temperature, obtainable in such a way, that the coating is performed with calibrated standard nucleic acids with addition of defined quantities of a carrier nucleic acids in a non-covalent manner at chemically or biochemically non-modified surfaces of the inner walls of reaction chambers.

2. Reaction chambers according to claim1, **characterized in that** they are comprised of glass or plastic vessels or of glass capillaries.

3. Reaction chambers according to claims 1 and 2, **characterized in that** DNA, RNA, synthetic equivalents of DNA and/or RNA, as well as dU-containing DNA are used as standard nucleic acids.

4. Reaction chambers according to claims 1 to 3, **characterized in that**
a) for the dilution of DNA standards, a DNA solution is used comprising a minimum sequence homology to the nucleic acid compound to be analyzed
b) a tRNA solution is used for the dilution of the RNA standards.

5. Reaction chambers according to claims 1 to 4, **characterized in that**, as the carrier nucleic acid, DNA of lamda phages is used, which previously is transferred into easily desorbable fragments of a mean length of about 1 - 2 kb by means of ultrasonic treatment.

6. A method for the production of reaction chambers according to claims 1 to 5, **characterized in that**, calibrated standard nucleic acids and defined quantities of carrier nucleic acids are directly aliquoted into reaction chambers suitable for enzymatic amplification, and are subsequently non-covalently adsorbed directly in the inner wall of the reaction chamber by means of freeze-drying or vacuum-centrifugating lyophilization.

7. Method according to claim 6, **characterized in that** plastic vessels or glass capillaries are coated.

8. Method according to claims 6 and 7, **characterized in that** DNA, RNA, synthetic equivalents and/or RNA, as well as dU-containing DNA are used as nucleic acids.

9. Method according to claims 6 to 8, **characterized in that**
a) for the dilution of DNA standards, a DNA solution is used comprising a minimum sequence homology to the nucleic acid compound to be analyzed, and
b) a tRNA solution is used for the dilution of the RNA standards.

10. Method according to claims 6 to 9, **characterized in that**, as the carrier nucleic acid, DNA of lamda phages is used, which previously is transferred into easily desorbable fragments of a mean length of about 1 - 2 kb by means of ultrasonic treatment.

11. Method according to claims 6 to 10, **characterized in that** corresponding reaction chambers are simultaneously coated with a plurality (at least two) of different analyte sequence-specific calibrated nucleic acids, if necessary, or a different cellular or organic origin or originating from different species.

12. Method according to claims 6 to 11, **characterized in that** the coating of at least 96 reaction chambers arranged in a microtiter format ensues with at least 12 x 8 sequence-specific standard nucleic acids of decreasing concentrations covering the entire expected concentration range of the analyte nucleic acid to be measured (highest concentration: A1 -12, lowest concentration: H1 - 12).

13. Method according to claims 6 to 12, **characterized in that** the coated reactions chambers are closed standing upright in an appropriate carrier box receiving at least 96 vessels.

14. Method according to claims 6 to 13, wherein, apart from the calibrated nucleic acids, at least two specific marked or unmarked oligonucleotides acting as primers or probes, the carrier nucleic acid and further reaction components required for the enzymatic amplification are contained in the reaction chambers in a lyophilized form, or specifically marked or unmarked oligonucleotides acting as primers or probes, the carrier nucleic acid and further reaction components required for the enzymatic amplification are contained in separate vessels without nucleic acid standard in a lyophilized form.

15. Use of the reaction chambers coated with nucleic acids according to claims 1 through 5 in test kits for the detection of selected nucleic acids in biological substances.

16. Use according to claim 15, **characterized in that** said test kits are comprised of at least one ZeptoStrip (octet strip of closed reaction chambers coated with eight different nucleic acid concentrations) closed with a film / foil, of at least two oligonucleotides, as well as one carrier nucleic acid.

## Revendications

1. Espaces de réaction revêtus des acides nucléiques produits d'une manière native, synthétique ou enzymatique stockable à température ambiante sans perte de qualité, procurables en ce que le revêtement est effectué avec des acides nucléiques standards calibrés en ajoutant des quantités définies d'un acide nucléique porteur d'une manière non covalente sur des surfaces non modifiées chimiquement ou biochimiquement de la paroi interne des espaces de réaction par séchage indulgent.

2. Espaces de réaction selon la revendication 1, **caractérisés en ce qu'**ils comportent des récipients en verre ou en plastique ou des capillaires en verre.

3. Espaces de réaction selon les revendications 1 et 2, **caractérisés en ce que** ADN, ARN, des produits de synthèse de ADN et / ou ARN ainsi que ADN contenant dU sont utilisés comme des acides nucléiques standards.

4. Espaces de réaction selon les revendications 1 à 3, **caractérisés en ce que**
a) pour la dilution des standards ADN une solution ADN est utilisée montrant une homologie de séquence minimale avec le mélange de l'acide nucléique à analyser
b) pour la dilution des standards ARN une solution tARN est utilisée.

5. Espaces de réaction selon les revendications 1 à 4, **caractérisés en ce que** comme acide nucléique porteur ADN de lambda-phage est utilisé qui est préalablement transformé au moyen d'un traitement ultrason dans des fragments facilement désorbables d'une longueur moyenne de 1 à 2 kb environ.

6. Procédure pour la production des espaces de réaction selon les revendications 1 à 5, **caractérisée en ce que** des acides nucléiques standards calibrés et des quantités définies de l'acide nucléique porteur ajouté sont aliquotés directement dans des espaces de réaction aptes à l'amplification enzymatique et sont ensuite adsorbés d'une manière non covalente directement à la paroi interne de l'espace de réaction par lyophilisation au moyen de cryodessication ou centrifugation sous vide.

7. Procédure selon la revendication 6, **caractérisée en ce que** des récipients en plastiques et/ou des capillaires en verre sont revêtus.

8. Procédure selon les revendications 6 et 7, **caractérisée en ce que** ADN, ARN, des produits de synthèse de ADN et / ou ARN ainsi que ADN contenant dU sont utilisés comme des acides nucléiques.

9. Procédure selon les revendications 6 à 8, **caractérisée en ce que**
a) pour la dilution des standards ADN une solution ADN est utilisée montrant une homologie de séquence minimale avec le mélange de l'acide nucléique à analyser et
b) pour la dilution des standards ARN une solution tARN est utilisée.

10. Procédure selon les revendications 6 à 9, **caractérisée en ce que** comme acide nucléique porteur ADN de lambda-phage est utilisé qui est préalablement transformé au moyen d'un traitement ultrason dans des fragments facilement désorbables d'une longueur moyenne de 1 à 2 kb environ.

11. Procédure selon les revendications 6 à 10, **caractérisé en ce que** des espaces de réaction correspondants sont revêtus simultanément avec une pluralité (au moins 2) des acides nucléiques divers, calibrés, par séquence analytique, le cas échéant de l'origine différente cellulaire ou organique et/ou issus des espèces différentes.

12. Procédure selon les revendications 6 à 11, **caractérisée en ce que** le revêtement d'au moins 96 espaces de réaction disposés en format plaques microtitres est effectué avec des concentrations diminuant avec au moins 12 x 8 acides nucléiques standards par séquence, et comprenant la plage totale de concentration attendue de l'acide nucléique analyte à mesurer (concentration maximale : A1-12, concentration minimale: H1-12).

13. Procédure selon les revendication 6 à 12, **caractérisée en ce que** les espaces de réaction revêtus sont fermés placés debout dans une boîte porteuse appropriée recueillant au moins 96 récipients.

14. Procédure selon les revendications 6 à 13, contenant dans les espaces de réaction outre les acides nucléiques calibrés au moins deux oligonucléotides spécifiques marqués ou non marqués agissant comme des primers et/ou des sondes, l'acide nucléique porteur ainsi que des autres composants de réaction nécessaires pour l'amplification enzymatique sous forme lyophilisée, ou des oligonucléotides spécifiques marqués ou non marqués agissant comme des primers et/ou des sondes, l'acide nucléique porteur ainsi que des autres composants de réaction nécessaires pour l'amplification enzymatique dans des récipients séparés sans standard de l'acide nucléique sous forme lyophilisée.

15. Utilisation des espaces de réaction revêtus de l'acide nucléique selon les revendications 1 à 5 dans des kits d'essai pour déterminer des acides nucléiques choisis dans des substances biologiques.

16. Utilisation selon la revendication 15, **caractérisée en ce que** les kits d'essai comprennent au moins un ZeptoStrip (strip de 8 d'espaces de réaction fermés et revêtus de 8 concentrations de l'acide nucléique différentes) fermé par une feuille, au moins deux oligonucléotides ainsi qu'un acide nucléique porteur.
